(19) Europäisches Patentamt
European Patent Office
Office européen des brevets

(11) Publication number: **0 075 451**
**B1**

(12) **EUROPEAN PATENT SPECIFICATION**

(45) Date of publication of patent specification: **16.07.86**

(51) Int. Cl.$^4$: **C 07 D 501/26,**
C 07 D 501/30, A 61 K 31/545

(21) Application number: **82304895.4**

(22) Date of filing: **16.09.82**

(54) **Cephalosporin derivatives.**

(30) Priority: **22.09.81 JP 149868/81**
**22.09.81 JP 149870/81**

(43) Date of publication of application:
**30.03.83 Bulletin 83/13**

(45) Publication of the grant of the patent:
**16.07.86 Bulletin 86/29**

(84) Designated Contracting States:
**BE CH DE FR GB IT LI SE**

(56) References cited:
**US-A-3 216 999**
**US-A-3 270 009**

(73) Proprietor: **KUREHA KAGAKU KOGYO KABUSHIKI KAISHA**
**9-11 Horidome-cho 1-chome Nihonbashi**
**Chuo-ku**
**Tokyo (JP)**

(72) Inventor: **Muto, Shigeaki**
**3-23-2 Higashi-Horikiri**
**Katsushika-ku Tokyo (JP)**
Inventor: **Niimura, Kouichi**
**31-201 Shinsayama-Haitsu**
**63 Aoyagi Sayama-shi Saitama-ken (JP)**
Inventor: **Ando, Takao**
**No. 24 Daiichi-Kurehasoh**
**3-10-13 Nerima Nerima-ku Tokyo (JP)**
Inventor: **Fujii, Masahiko**
**202 Mezon Shirayuri 1-6-11 Nakaizumi**
**Komae-shi Tokyo (JP)**
Inventor: **Furusho, Takao**
**1-6-13 Asahi-machi**
**Machida-shi Tokyo (JP)**
Inventor: **Yoshikumi, Chikao**
**2-19-46 Higashi**
**Kunitachi-shi Tokyo (JP)**
Inventor: **Kanno, Akihiko**
**5-31-1 Matsubara**
**Setagaya-ku Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 075 451 B1**

(74) Representative: **Myerscough, Philip Boyd et al**
**J.A.Kemp & Co. 14, South Square Gray's Inn**
**London, WC1R 5EU (GB)**

**Description**

The present invention relates to cephalosporin compounds, their preparation and pharmaceutical compositions containing them.

Cephalosporins are well known as excellent antibiotics due to their selective toxicity to bacteria. However, cephalosporin antibiotics have a serious defect, that is they may disturb the beneficial bacterial colonies ordinarily present in living bodies, particularly the intestinal bacterial colonies, since the cephalosporins may be also antibacterially active against the beneficial bacteria. This defect is very serious when such an antibiotic is orally administered. As a result, "microbisme selectioné et substitué" is caused resulting in colitis and diarrhoea.

The present invention is concerned with antibiotics without this defect. In particular, the present invention relates to a compound obtained by chemically modifying a cephalosporin, antibacterial activity being reduced by such a chemical modification but recovered when the compound is absorbed from the intestinal tract. The cephalosporins of the invention can be formulated into pharmaceutical compositions to provide a medicine exhibiting an antibacterial activity similar to a cephalosporin antibiotic in living body.

Compounds of the present invention (hereinafter referred to as the present compound) have the general formula (I):

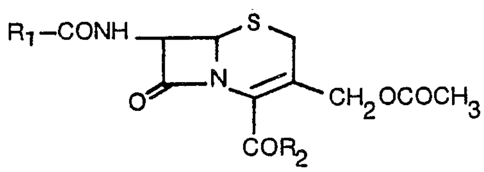

(I)

wherein $R_1$ is

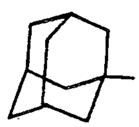

or

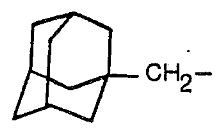

;

and $R_2$ is an alkoxy group having 1 to 4 carbon atoms,

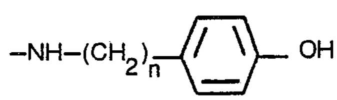

wherein n is 0, 1 or 2; or

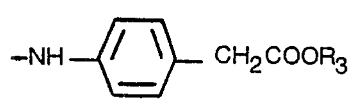

wherein $R_3$ is a hydrogen atom, an alkyl group having 1 to 4 carbon atoms or a pharmaceutically acceptable cation, preferably an alkali metal cation. However, the present compound may be in a form other than an alkali metal salt, for example an alkaline earth metal salt, aluminum salt or ammonium salt. The cephalosporins of formula (I), and indeed all cephalosporins described and illustrated by formulae herein, have the usual absolute cephalosporin stereochemistry of 6R:7R.

The present compound is derived from a cephalosporin antibiotic by a chemical modification. It is absorbed into a living body without affecting the bacterial colonies ordinarily present in living bodies and shows an antibacterial activity when entering into blood. Therefore, the present compound is an antibiotic of a type quite different from the conventional cephalosporin antibiotics.

The present compound may be synthesized by the following process.

7-Aminocephalosporanic acid having the general formula (II-a);

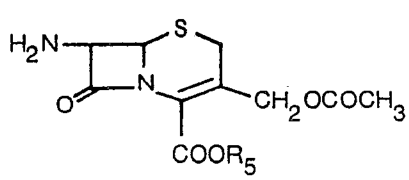

(II—a)

wherein $R_5$ is a hydrogen atom, an alkyl group having 1 to 4 carbon atoms or a pharmaceutically acceptable cation is reacted with an acid chloride of the formula (III);

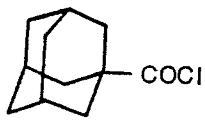 or 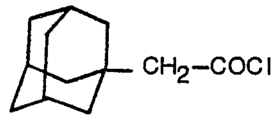 (III)

Generally the 7-aminocephalosporanic acid is added drop by drop in a mixed solvent of water and acetone in the presence of alkali to an acetone solution of the acid chloride. The whole system is brought into reaction for 0.5 to 48 hours at a temperature of −30 to +40°C. After the reaction is over, the present compound can be collected by conventional methods such as extraction with a solvent, washing with a solvent and recrystallization. When $R_5$ is an alkyl group having 1 to 4 carbon atoms, a compound of the invention of formula (I) in which $R_2$ is an alkoxy group having 1 to 4 carbon atoms is obtained.

However, the thus-obtained cephalosporin derivative may be dissolved in an organic solvent, for example, benzene, tetrahydrofuran, dimethylformamide, chloroform, pyridine, dicyclohexylamine, acetone, triethylamine, chloromethane, dioxane, methanol, ethanol, water, ether and the like. It is preferable to add an activating agent such as carbodiimide, ethyl chloroformate, oxalyl chloride and the like. Then into the thus-prepared solution, an amine compound having the general formula (IV);

$$NH_2—R_6 \qquad (IV)$$

wherein $R_6$ is

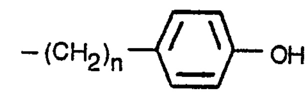 or 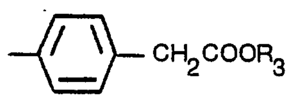

wherein n and $R_3$ are the same meanings as above, is added. The whole system is brought into reaction at the temperature of −30 to +50°C for 0.5 to 48 hours. After the reaction is over, the cephalosporin of the invention of formula (I) in which $R_2$ is the same as $R_6$ above can be collected by conventional methods such as extraction with a solvent, washing with a solvent, recrystallization and the like.

As seen from Examples described below, the present compounds have low toxicity and exhibit an antibacterial activity in a living body without affecting the intestinal bacterial colonies.

The present compound can be useful in the same field as the conventional cephalosporin antibiotics since the present compound is transformed into a cephalosporin antibiotic in a living body.

The present compound can be used in a dosage unit form such as a drug or a pharmaceutical composition. The composition may contain 0.01 to 99.5% by weight, generally 0.1 to 90% by weight of the present compound as an active ingredient.

The pharmaceutical composition contains a pharmaceutically acceptable carrier, diluent or adjuvant as well as at least one of the present compound. Further, the composition may contain a filler, extender, binder, wetting agent, disintegrant, retarder of dissolution, accelerator of reabsorption, adhesive carrier and/or lubricant, for example, starch, mannitol, silicic acid, cellulose derivative, gelatin, alginate, glycerol, agar, calcium carbonate, sodium hydrogen carbonate, paraffin, quatarnary ammonium compound, glycerol monostearate, kaolin, bentonite, talc, potassium stearate or polyethylene glycol.

The pharmaceutical composition may be administered orally or rectally or by injection. The dosage form for oral administration may be a tablet, capsule, powder, granule, pill or ampoule. The composition may be also in the form of pharmaceutically acceptable emulsion, solution or suspension.

A syrup or elixir may contain an inert diluent such as water or paraffin and may be used as a liquid composition suitable for oral administration. These composition may contain an adjuvant such as a wetting agent, edulcorant or seasoning agent.

A suppository containing the present compound as an active ingredient may contain polyethylene glycol and/or a fatty acid or ester thereof.

The pharmaceutical composition for injection may be a sterilized aqueous or non-aqueous solution, suspension or emulsion and may contain, for example, propylene glycol, polyethylene glycol or olive oil.

The present compound may be useful for the same as the conventional cephalosporin antibiotics and effective in treating an infectious disease due to bacteria such as Streptococcus, Pneumococcus, Gonococcus, diphteria bacillus, Staphylococcus, Spirochaeta, Actinomyces, Shigella, E. coli, Myxomycetes, Enterecoccus and Meningococcus. The conditions which can be treated with the present compound are exemplified as follows; tonsilitis, pharyngitis, laryngitis, wound, burn, postoperative secondary infection, lymphadenitis, septicemia, bacterial endocarditis, pneumonia, pulmonary suppuration, bronchitis, scarlet fever, gonorrhea, cystitis, pyothorax, urethritis, bacterial dysentery, meningitis, diphtheria, otitis media, carbuncle and actinomycosis.

The dose of the drug or the pharmaceutical composition of the present compound may depend on the degree of the infection and the condition of the patient, and generally a dose of 0.1 to 10 g may be administered to an adult patient per one day, divided into several times.

The following Examples illustrate the invention. A Reference Example is provided.

Reference Example

Synthesis of the compound represented by the following formula;

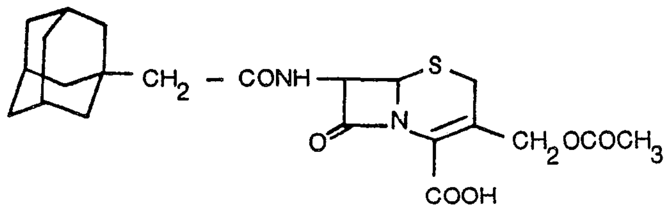

Into a solution of 2.72 g of 7-aminocephalosporanic acid and 1.68 g of sodium hydrogen carbonate in a mixed solvent of 30 ml of water and 20 ml of acetone, a solution of 2.13 g of 1-adamantaneacetyl chloride in 5 ml of acetone was added drop by drop while stirring the mixed solvent at 0°C. After stirring the reaction mixture for one hour at 0°C and further stirring for one hour at room temperature, it was left for a night at room temperature, and then the pH of the reaction mixture was adjusted to 4 by 1N-hydrochloric acid to educe the thus formed crystals. The crystals were extracted with 200 ml of ethyl acetate, and after washing the extract two times with water and drying on anhydrous magnesium sulfate, the solvent was distilled off from the dried extract. After recrystallizing the residue from a mixed solvent of ethyl acetate and n-hexane, 2.77 g of a white powder, 7-(adamantane-1-acetamido)cephalosporanic acid, was obtained with a yield of 62%.

The characteristics of the present compound thus obtained were as follows:

(1) melting point; 201—204°C,

(2) elementary analysis;

| | C(%) | H(%) | N(%) |
|---|---|---|---|
| theoretical: | 59.19 | 6.28 | 6.28 |
| experimental: | 59.11 | 6.29 | 6.18 |

(3) infrared absorption bands (KBr method); shown in Figure 1.

Example 1

Synthesis of the compound represented by the following formula;

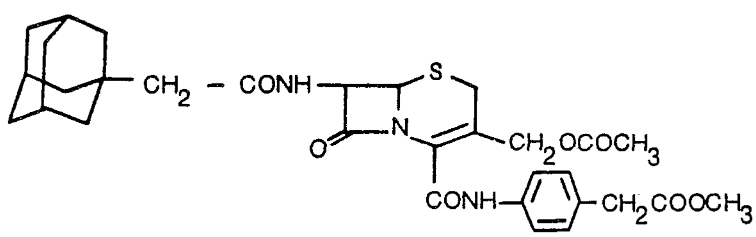

2.24 g of 7-(adamantane-1-acetamido)cephalosporanic acid, 0.83 g of methyl 4-aminophenylacetate and 1.05 g of N,N'-dicyclohexylcarbodiimide were dissolved in 30 ml of tetrahydrofuran. The thus-prepared solution was stirred at 25°C for 24 hours. The crystals formed in the reaction mixture were filtered off and washed with 30 ml of tetrahydrofuran. After recrystallizing the residue from ethanol, 1.2 g of white powdery crystals, N-(4-carbomethylphenyl)-7-(adamantane-1-acetamido)cephalosporanoamide, were obtained with a yield of 40%.

The characteristics of the present compound thus obtained were as follows:

(1) melting point; 190—192°C,

(2) elementary analysis;

| | C(%) | H(%) | N(%) |
|---|---|---|---|
| theoretical: | 62.52 | 6.22 | 7.06 |
| experimental: | 62.5 | 6.2 | 7.1 |

(3) infrared absorption bands (KBr method); $\nu_{max}$ ($cm^{-1}$): 2930, 1785, 1740, 1663, 1540, 1230 refer to Figure 2

(4) ultraviolet absorption bands in acetonitrile; $\lambda_{max}$ (nm): 223, 269.

Example 2

Synthesis of the compound represented by the following formula;

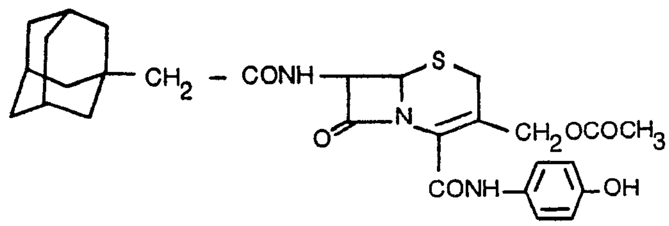

4.48 g of 7-(adamantane-1-acetamido)cephalosporanic acid, 1.09 g of 4-aminophenol and 2.10 g of N,N'-dicyclohexylcarbodiimide were dissolved into 60 ml of tetrahydrofuran. The thus-prepared solution was stirred at 15°C for 24 hours. The crystals formed in the reaction mixture were filtered off and washed with 30 ml of tetrahydrofuran. After recrystallizing the residue from ethanol, 2.1 g of white powdery crystals, N-(4-hydroxyphenyl)-7-(adamantane-1-acetamido)cephalosporanoamide, were obtained with a yield of 39%.

The characteristics of the present compound thus obtained were as follows:

(1) melting point; 190—191°C,

(2) elementary analysis;

| | C(%) | H(%) | N(%) |
|---|---|---|---|
| theoretical: | 65.52 | 6.22 | 7.06 |
| experimental: | 62.5 | 6.2 | 7.1 |

(3) infrared absorption bands (KBr method); $v_{max}$ ($cm^{-1}$): 2930, 1788, 1747, 1663, 1521, 1227 refer to Figure 3

(4) ultraviolet absorption bands in acetonitrile; $\lambda_{max}$ (nm): 237, 272.

Example 3

Synthesis of the compound represented by the following formula;

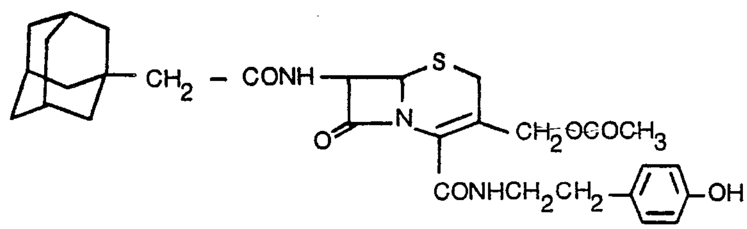

4.48 g of 7-(adamantane-1-acetamido)cephalosporanic acid, 1.37 g of quillamine and 2.10 g of N,N'-dicyclohexylcarbodimide were dissolved in 70 ml of tetrahydrofuran. The thus-prepared solution was stirred at 10°C for 34 hours. The crystals formed in the reaction mixture were filtered off and washed with 30 ml of tetrahydrofuran. After recrystallizing the residue from a mixed solvent of dimethylformamide and ethyl acetate, 2.9 g of pale-yellow powder crystals, N-[beta-(4-hydroxyphenyl)ethyl]-7-(adamantane-1-acetamido)cephalosporanoamide, were obtained with a yield of 51%.

The characteristics of the present compound thus obtained were as follows:

(1) melting point; 154—156°C (decomposition),

(2) elementary analysis;

| | C(%) | H(%) | N(%) |
|---|---|---|---|
| theoretical: | 63.49 | 6.53 | 7.41 |
| experimental: | 63.6 | 6.5 | 7.4 |

(3) infrared absorption bands (KBr method); $v_{max}$ ($cm^{-1}$): 2930, 1766, 1735, 1646, 1520, 1240 refer to Figure 4,

(4) ultraviolet absorption bands in acetonitrile; $\lambda_{max}$ (nm): 223, 265

Toxicological and pharmacological activities of the present compounds

Example 4

Acute toxicity of the present compounds were determined as follows.

Each of the present compounds was dispersed in a physiological saline solution. The dispersion was administered to an ICR—JCL mouse orally by a stomach sonde or intraperitoneally by injection at a predetermined amount.

After administration, the intoxication symptoms were continuously observed for a week and both survival and dead mice were autopsied. $LD_{50}$ value was obtained from the cumulative mortality of the treated mice by applying the data to the Litchfield-Wilcoxon's graphical method. All of the present compounds gave $LD_{50}$ value of more than 10 g/kg in both oral and intraperitoneal administrations. The $LD_{50}$ value of cephalotin sodium as a comparative antibiotic is about 5 g/kg.

These results show that the present compound is a safe substance having a low toxicity.

Example 5

Effect of the present compounds on the intestinal bacterial colonies was examined.

Each of the present compounds was orally administered to mice (one group consisting of five 6-week old female ICR mice) for two consecutive days at a dose of 500 mg/kg/day.

Before and on the first day after the administration, feces of each mouse were collected and diluted with an anaerobic diluent (phosphoric buffer solution) of 100 times volume and ground. 0.1 ml of the diluted and ground feces was smeared on each culture medium shown in Table 1 and cultured aerobically or anaerobically (according to the anaerobic glove box method) under the conditions shown in Table 1. The number of each bacterium shown in Table 1 was counted.

The results are shown in Table 2.

As seen from Table 2, the number of *Escherichia coli* showed no remarkable change as compared to that before administration in the case of each of the present compounds, while that number increased in the case of cephalotin as a comparative antibiotic. Further, the number of *Lactobacillus acidophilus* decreased in the case of cephalotin while such a decrease was not observed in the case of the present compounds.

These results show that the present compounds do not affect the intestinal bacterial colonies in living bodies.

TABLE 1
Culture medium and culture condition of bacteria

| Bacterium | Culture medium | Culture condition |
|---|---|---|
| *Escherichia coli* | DHL agar | aerobic, 37°C, one day |
| *Pseudomonas aeruginosa* | NAC agar | aerobic, 37°C, one day |
| *Streptococcus* spp. | TATAC agar | aerobic, 37°C, one day |
| *Lactobacillus acidophilus* | LBS agar | anaerobic, 37°C, five days |
| *Lactobacillus bifidus* | BS agar | anaerobic, 37°C, five days |
| *Bacteroides* | NBGT agar | anaerobic, 37°C, five days |

TABLE 2

| Example No. | Logarithmic value of the number of bacterial cells per one gram of feces | | | | | |
|---|---|---|---|---|---|---|
| | *E. coli* | *Ps. aeruqinosa* | *Strept.* spp. | *L. acidophilus* | *L. bifidus* | *Bacteroides* |
| 1 | 6.6 | <3.0 | 6.8 | 9.0 | 8.3 | 8.6 |
| 2 | 6.5 | <3.0 | 6.7 | 8.9 | 8.6 | 8.7 |
| 3 | 6.4 | <3.0 | 6.8 | 8.9 | 8.5 | 8.7 |
| before administration | 6.4 | <3.0 | 6.8 | 9.0 | 8.4 | 8.3 |
| Cephalotin | 9.9 | <3.0 | 9.0 | 4.1 | 9.2 | 7.2 |

Example 6

Antibacterial activity of the present compounds was examined as follows.

Antibacterial activity of each of the present compounds was examined against the following two bacteria according to the standard method of Japan Society of Chemotherapy:

*Escherichia coli* IFO 12734 and

*Staphylococcus aureus* IAM 1011

Each bacterial strain was inoculated into the Mueller-Hinton's culture medium and cultured at 37°C for 18 to 48 hours. The culture medium was diluted so as to contain $1 \times 10^6$ cells of the bacterial per one ml, and the obtained medium was used as the bacterial specimen.

Agar plates were prepared by adding one part by weight of each solution of the present compounds having a predetermined concentration to nine parts by weight of Mueller-Hinton's culture medium.

A loopful amount of the bacterial specimen prepared above was smeared to make a streak of about 2 cm on each agar plate and cultured at 37°C for 18 to 24 hours. The minimum concentration for completely inhibiting proliferation of the bacteria (referred to as MIC) was determined.

The results are shown in Table 3.

TABLE 3

| Example No. | MIC | |
|---|---|---|
| | *E. coli* | *Staph. aureus* |
| 1 | 100≤ | 100≤ |
| 2 | 100≤ | 100≤ |
| 3 | 100≤ | 1.56 |

Example 7

The following experiment was carried out in order to prove that the present compound is activated in a living body.

As an enzyme for activating metabolism, a rat liver homogenate (S-9, manufactured by Oriental Yeast Company, Japan) was used in the following composition per one ml (hereinafter referred to as S-9 mix).

| | |
|---|---|
| S-9 | 0.5 ml |
| KCl | 3.3 µmol |
| $MgCl_2 \cdot 6H_2O$ | 8 µmol |
| Glucose-6-phosphate | 5 µmol |
| NADH | 4 µmol |
| NADPA | 4 µmol |
| 0.2 M phosphoric buffer solution (pH 7.4) | 0.5 ml |

0.1 ml of each solution of the present compounds at a concentration of various value was mixed with 0.9 ml of S-9 mix or 0.9 ml of 0.1 M phosphoric buffer solution (as a control) and the obtained mixture was incubated at 37°C for 20 min with shaking.

*Staphylococcus aureus* IAM 1011 was inoculated into a Mueller-Hinton's culture medium and cultured at 37°C for 18 hours. The culture medium was adjusted to a cell concentration of $1 \times 10^8$ per one ml and mixed with 50 times by volume of Mueller-Hinton's agar culture medium to obtain an agar plate.

A penicillin cup of 8 mm in diameter was placed on the agar plate prepared above, and into the cup 0.1 ml of the mixture was introduced and allowed to stand at 4°C for 2 hours and then cultured at 37°C for 18 hours to measure the diameter of a circle in which the proliferation of bacteria was inhibited (proliferation-inhibiting circle). The results are shown in Table 4. In Table 4, the proliferation-inhibiting index is shown with the ratio (%) of the diameter of the proliferation-inhibiting circle obtained by using each of the present compounds to that obtained by using the comparative compound.

| Index | % |
|---|---|
| — | 0 |
| ± | 0—1 |
| + | 1—33 |
| ++ | 33—66 |
| +++ | 66—100 |

TABLE 4

| Example No. | Proliferation-inhibiting index (%) | |
|---|---|---|
| | Before adding S-9 mix | After adding S-9 mix |
| 1 | — | + |
| 2 | — | + |
| 3 | + | +++ |

As seen from Table 4, the antibacterial activity of the present compound is activated by an enzyme in a living body, although it itself shows a low antibacterial activity in the absence of an activating enzyme.

Example 8

Effect of the present compounds on the infection was examined.

*Escherichia coli* IFO 12734 (1.4 × $10^8$) was inoculated intraperitoneally to ddY-SPF mice (a group consisting of 20 mice). Just after and at 4 hours after the infection, each of the present compounds was administered orally at a dose of 500 mg/kg and the mortality of the mice due to the infection was observed for 7 days. More than 35% of the mice administered with the present compound survived even on the 7th day after the infection, while all mice without the administration with the present compound died on the 2nd day after infection.

The results show that the present compound is an effective medicine for oral administration against an infectious disease.

Manufacture of the Pharmaceutical Preparations:

Example 9

(1) Tablet

A tablet was prepared by a following composition in one table of 200 mg;

| | |
|---|---|
| the present compound of Example 1 | 175 mg |
| lactose | 16 mg |
| starch | 5 mg |
| hydroxypropylcellulose | 3 mg |
| magnesium stearate | 1 mg |

The present compound and lactose were mixed and then an aqueous solution of hydroxypropylcellulose was admixed, and the mixture was kneaded, dried and pulverized. Then magnesium stearate dispersed previously into starch was admixed and the mixture was made into a tablet by the conventional method for tabletting.

(2) *Granule*

A granule was prepared by a following composition;

| | |
|---|---|
| the present invention of Example 2 | 176 mg |
| lactose | 16 mg |
| starch | 4 mg |
| hydroxypropylcellulose | 4 mg |

The present compound, starch and lactose were mixed, and an aqueous solution of hydroxypropylcellulose was admixed and the mixture was dried and pulverized. The pulverized material was sifted by 12 to 48 mesh sieves to obtain a granule.

**Claims**

1. A cephalosporin derivative having the general formula (I):

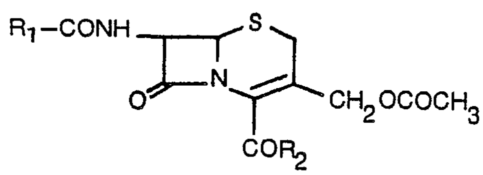

(I)

wherein $R_1$ is

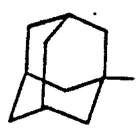

or

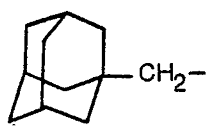

;

$R_2$ is an alkoxy group having 1 to 4 carbon atoms,

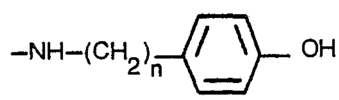

wherein n is 0, 1 or 2 or

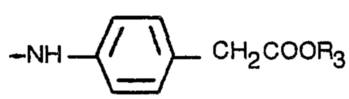

wherein $R_3$ is a hydrogen atom, an alkyl group having 1 to 4 carbon atoms or a pharmaceutically acceptable cation.

2. A derivative according to claim 1, which is N-(4-carbomethoxymethylphenyl)-7-(adamantane-1-acetamido)cephalosporanoamide.

3. A derivative according to claim 1, which is N-(4-hydroxyphenyl)-7-(adamantane-1-acetamido)cephalosporanoamide.

4. A derivative according to claim 1, which is N-[beta-(4-hydroxyphenyl)ethyl]-7-(adamantane-1-acetamido)cephalosporanoamide.

5. A process for the preparation of a cephalosporin derivative as claimed in claim 1 in which $R_2$ is the alkoxy group, which process comprises reacting a 7-aminocephalosporanic acid having the general formula (II):

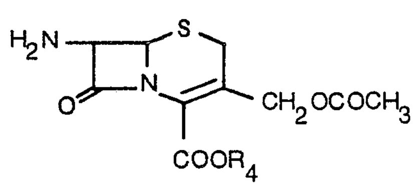

(II)

wherein $R_4$ is an alkyl group having 1 to 4 carbon atoms, with an acid chloride of formula (III):

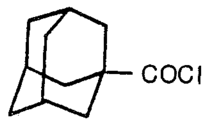

or

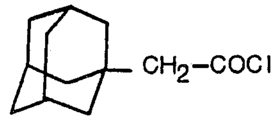

(III)

for 0.5 to 48 hours at a temperature of −30 to 40°C.

6. A process for the preparation of a cephalosporin derivative as claimed in claim 1 in which $R_2$ is other than the alkoxy group, which process comprises reacting a 7-aminocephalosporanic acid having the general formula (II-a):

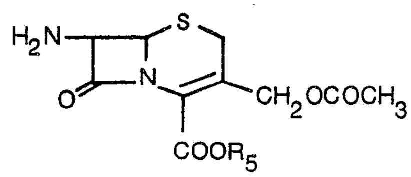

(II–a)

wherein $R_5$ is a hydrogen atom, an alkyl group having 1 to 4 carbon atoms or a pharmaceutically acceptable cation, with an acid chloride of (III) as depicted in claim 5 for 0.5 to 48 hours at a temperature of −30 to 40°C and reacting the cephalosporin derivative thus obtained with an amine of the general formula (IV):

$$NH_2—R_6 \qquad (IV)$$

wherein $R_6$ is

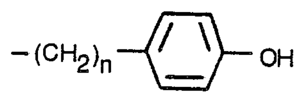

or

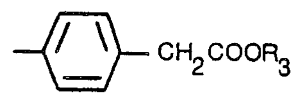

wherein n and $R_3$ are as defined in claim 1, for 0.5 to 48 hours at a temperature of −30 to 50°C to form a cephalosporin derivative as claimed in claim 1 wherein $R_2$ is the same as $R_6$ above.

7. A pharmaceutical composition comprising, as active ingredient, a cephalosporin derivative as claimed in any one of claims 1 to 4 or which has been prepared by a process as claimed in claim 5 or 6, together with a pharmaceutically acceptable carrier, diluent or adjuvant.

8. A pharmaceutical composition according to claim 7 in a unit dosage form.

**Patentansprüche**

1. Cephalosporinderivat mit der allgemeinen Formel (I):

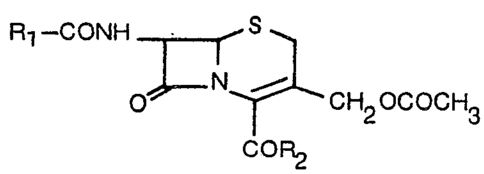

(I)

in der $R_1$

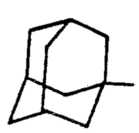

oder

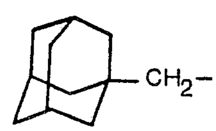

;

ist, $R_2$ eine Alkoxygruppe mit 1 bis 4 Kohlenstoffatomen,

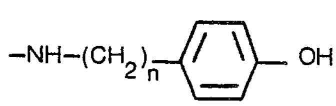

worin n 0, 1 oder 2 ist, oder

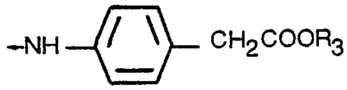

ist, worin $R_3$ ein Wasserstoffatom, eine Alkylgruppe mit 1 bis 4 Kohlenstoffatomen oder ein pharmazeutisch akzeptables Kation ist.

2. Derivat nach Anspruch 1, dadurch gekennzeichnet, daß es N-(4-Carbomethoxymethylphenyl)-7-(adamantan-1-acetamido)-cephalosporanoamid ist.

3. Derivat nach Anspruch 1, dadurch gekennzeichnet, daß es N-(4-Hydroxyphenyl)-7-(adamantan-1-acetamido)-cephalosporanoamid ist.

4. Derivat nach Anspruch 1, dadurch gekennzeichnet, daß es N-[beta-(4-Hydroxyphenyl)ethyl]-7-(adamantan-1-acetamido)-cephalosporanoamid ist.

5. Verfahren zur Herstellung eines Cephalosporinderivats gemäß Anspruch 1, wobei $R_2$ die Alkoxygruppe ist, dadurch gekennzeichnet, daß eine 7-Aminocephalosporansäure mit der allgemeinen Formel (II):

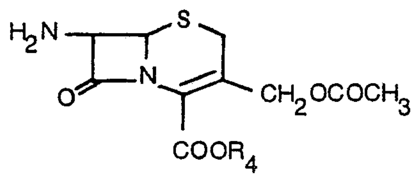

(II)

in der $R_4$ eine Alkylgruppe mit 1 bis 4 Kohlenstoffatomen ist, mit einem Säurechlorid der Formel (III):

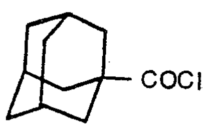

oder

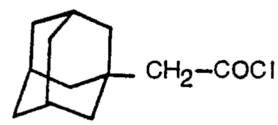

(III)

0,5 bis 48 Stunden bei einer Temperatur von −30 bis 40°C zur Reaktion gebracht wird.

6. Verfahren zur Herstellung eines Cephalosporinderivats gemäß Anspruch 1, wobei $R_2$ ein anderer Rest als die Alkoxygruppe ist, dadurch gekennzeichnet, daß eine 7-Aminocephalosporansäure mit der allgemeinen Formel (II-a):

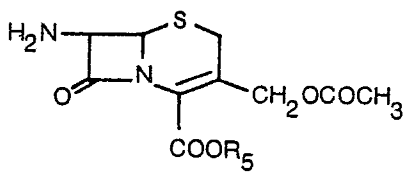

(II–a)

in der $R_5$ ein Wasserstoffatom, eine Alkylgruppe mit 1 bis 4 Kohlenstoffatomen oder ein pharmazeutisch akzeptables Kation ist, mit einem Säurechlorid gemäß (III), wie in Anspruch 5 wiedergegeben, 0,5 bis 48 Stunden bei einer Temperatur von −30 bis 40°C zur Reaktion gebracht wird und das so erhaltene Cephalosporinderivat mit einem Amin der allgemeinen Formel (IV):

$$NH_2—R_6 \qquad (IV),$$

in der $R_6$

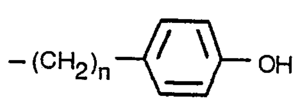

oder

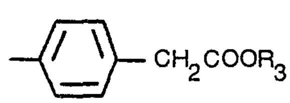

ist, wobei n und $R_3$ die in Anspruch 1 angegebene Bedeutung besitzen, 0,5 bis 48 Stunden bei einer Temperatur von −30 bis 50°C unter Bildung eines Cephalosporinderivats gemäß Anspruch 1, bei dem $R_2$ mit dem obigen $R_6$ übereinstimmt, zur Reaktion gebracht wird.

7. Pharmazeutische Zusammensetzung, die als aktiven Bestandteil ein Cephalosporinderivat gemäß einem der Ansprüche 1 bis 4 oder hergestellt nach einem Verfahren gemäß Anspruch 5 oder 6 zusammen mit einem pharmazeutisch akzeptablen Träger, Verdünnungsmittel oder Adjuvans enthält.

8. Pharmazeutische Zusammensetzung nach Anspruch 7 in Form einer Dosierungseinheit.

**Revendications**

1. Un dérivé de céphalosporine ayant la formule générale (I)

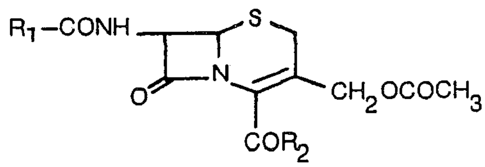

(I)

dans laquelle $R_1$ est

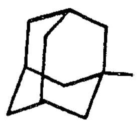

ou

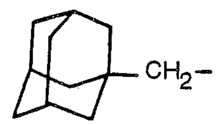

;

$R_2$ étant un groupe alkoxy ayant de 1 à 4 atomes de carbone,

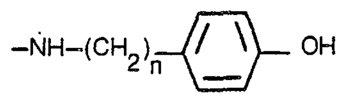

où n est 0, 1 ou 2 ou

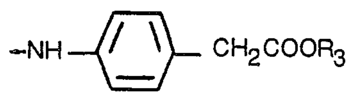

où $R_3$ est un atome d'hydrogène, un groupe alkyle ayant de 1 à 4 atomes de carbone ou un cation pharmaceutiquement acceptable.

2. Un dérivé selon la revendication 1, qui est une N-(4-carbométhoxyméthylphényl)-7-(adamantane-1-acétamido)céphalosporanoamide.

3. Un dérivé selon la revendication 1, qui est une N-(4-hydroxyphényl)-7-(adamantane-1-acétamido)céphalosporanoamide.

4. Un dérivé selon la revendication 1, qui est une N-[béta-(4-hydroxyphényl)éthyl]-7-(adamantane-1-acétamido)céphalosporanoamide.

5. Une méthode pour la préparation d'un dérivé de céphalosporine selon la revendication 1, dans lequel $R_2$ est le groupe alkoxy, la dite méthode comprenant la mise en réaction d'un acide 7-aminocéphalosporanique ayant la formule générale (II):

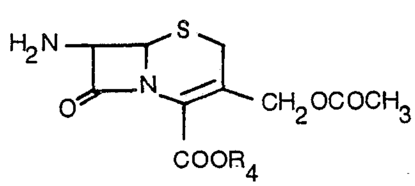

(II)

dans laquelle $R_4$ est un groupe alkyl ayant de 1 à 4 atomes de carbone, avec un chlorure d'acide de la formule (III):

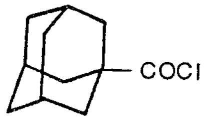

ou

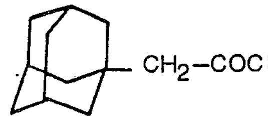

(III)

pendant une durée de 0,5 à 48 heures, à une température se situant entre −30 et 40°C.

6. Une méthode pour la préparation d'un dérivé de céphalosporine selon la revendication 1, dans lequel $R_2$ est autre que le groupe alkoxy, la dite méthode comprenant la mise en réaction d'un acide 7-aminocéphalosporanique ayant la formule générale (II-a):

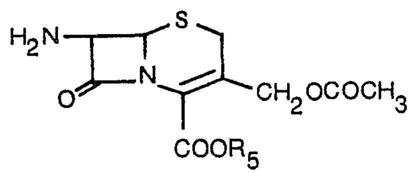

(II–a)

dans laquelle $R_5$ est un atome d'hydrogène, un groupe alkyl ayant de 1 à 4 atomes de carbone ou un cation pharmaceutiquement acceptable, avec un chlorure d'acide de la formule (III) comme décrit dans la revendication 5, pendant une durée de 0,5 à 48 heures à une température se situant entre −30 et 40°C, et la mise en réaction du dérivé de céphalosporine ainsi obtenu avec une amine de la formule générale (IV):

$$NH_2—R_6 \qquad (IV)$$

dans laquelle $R_6$ est

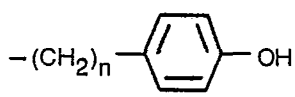

ou

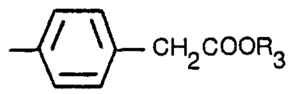

n et $R_3$ étant comme défini dans la revendication 1, pendant une durée de 0.5 à 48 heures à une température se situant entre −30 et 50°C, de manière à former un dérivé de céphalosporine selon la revendication 1, dans lequel $R_2$ est comme $R_6$ ci-dessus.

7. Une composition pharmaceutique comprenant comme ingrédient actif, un dérivé de céphalosporine comme revendiqué dans l'une quelconque des revendications 1 à 4 ou ayant été préparé par une méthode comme revendiqué dans la revendications 5 ou 6, conjointement avec un excipient, diluant ou adjuvant pharmaceutiquement acceptable.

8. Une composition pharmaceutique selon la revendication 7, sous forme d'unité de dosage.

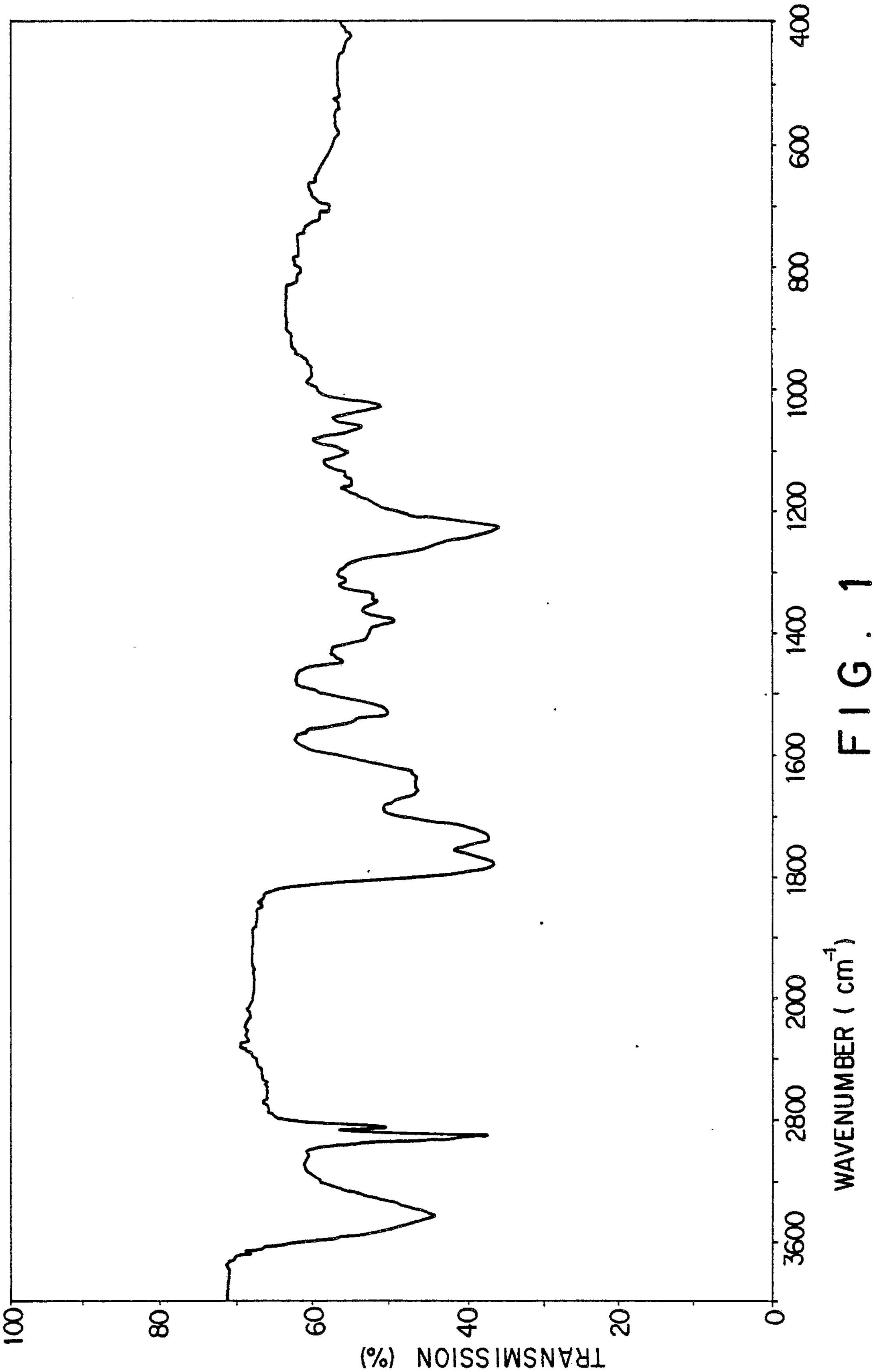
TRANSMISSION (%)
100
80
60
40
20
0
WAVENUMBER ( cm$^{-1}$ )
3600
2800
2000
1800
1600
1400
1200
1000
800
600
400

FIG. 1

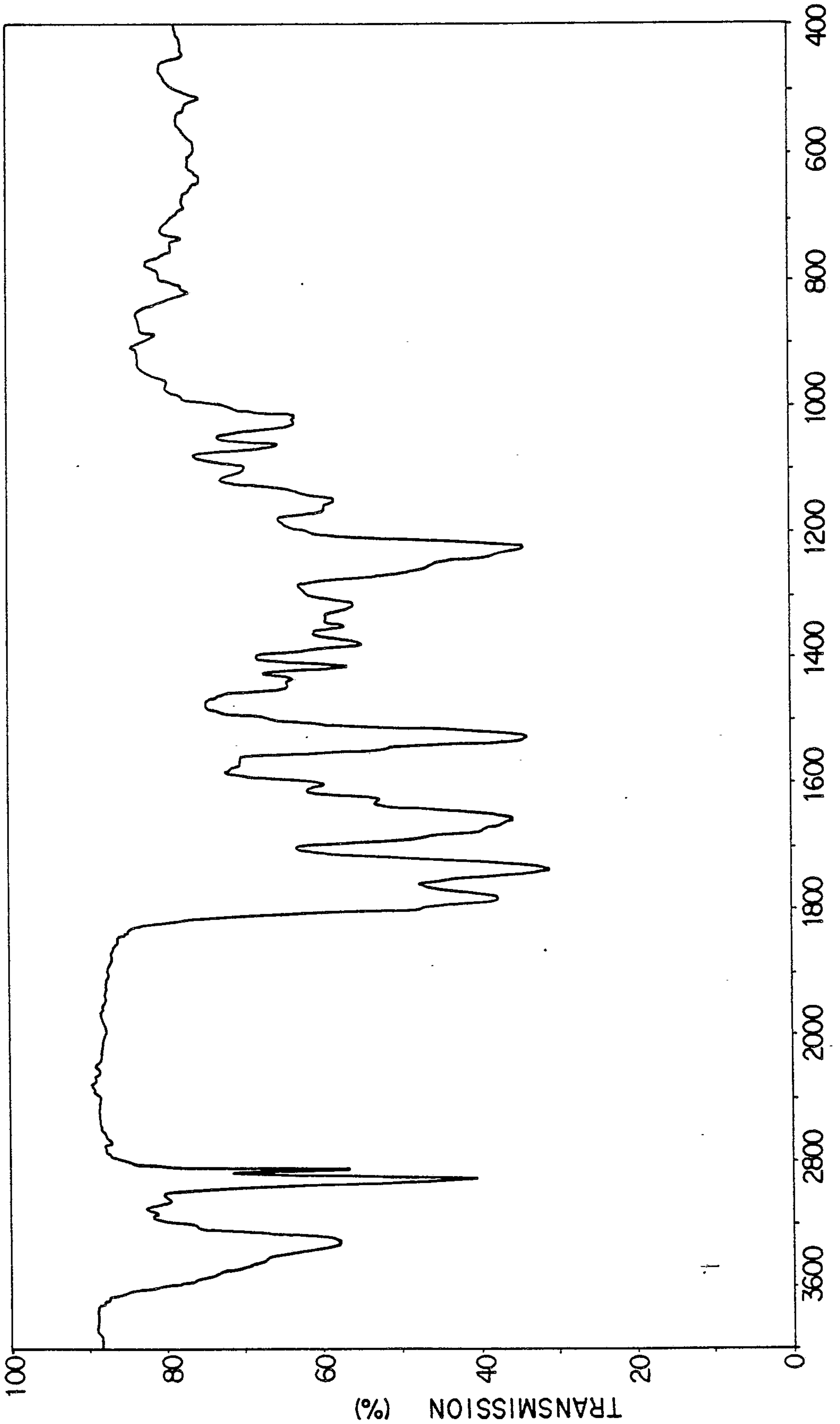
TRANSMISSION (%)
100
80
60
40
20
0
WAVENUMBER ( cm$^{-1}$)
3600
2800
2000
1800
1600
1400
1200
1000
800
600
400

F I G . 2

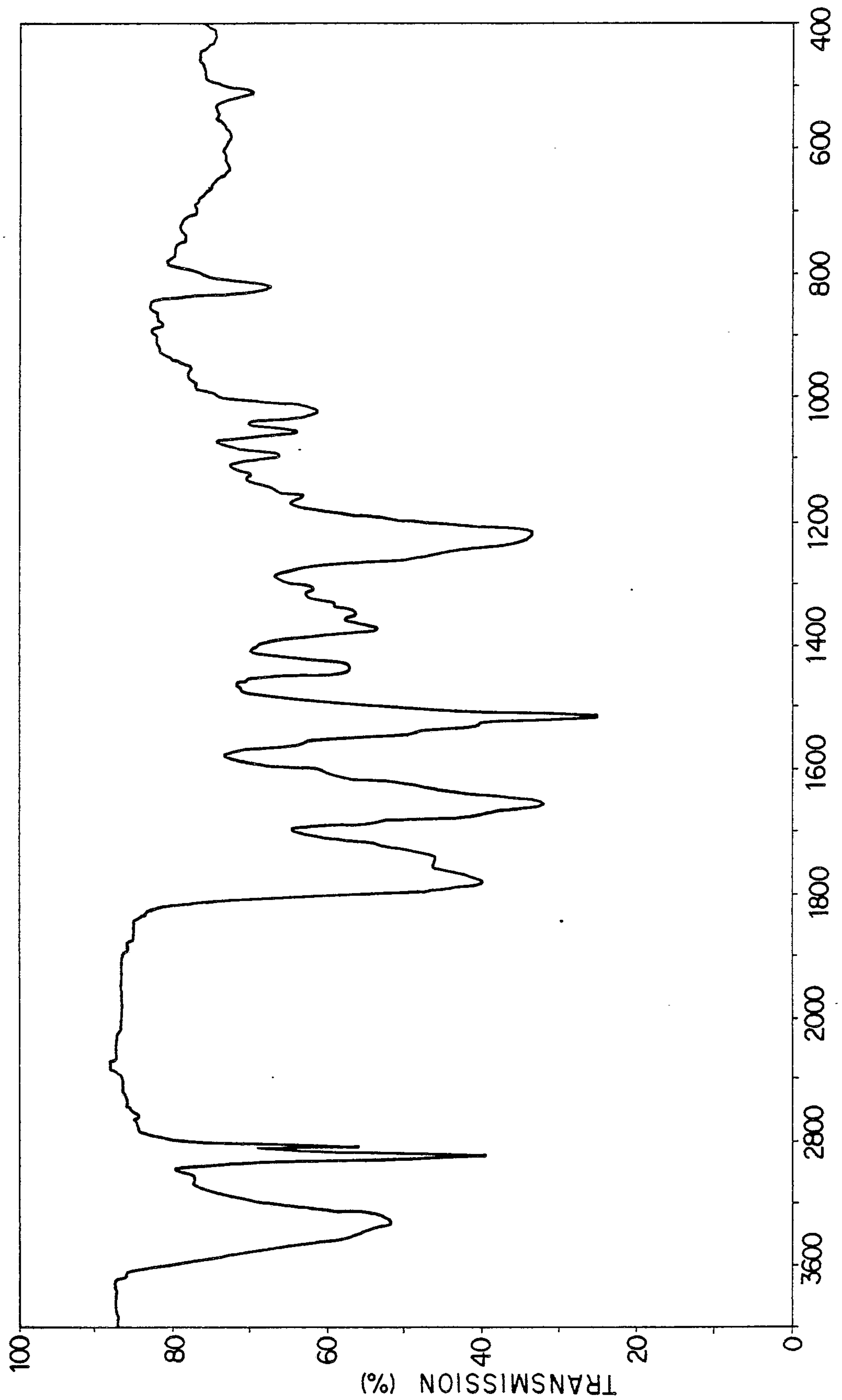
100
80
60
40
20
0
TRANSMISSION (%)
3600
2800
2000
1800
1600
1400
1200
1000
800
600
400
WAVENUMBER ( $cm^{-1}$ )

FIG. 3

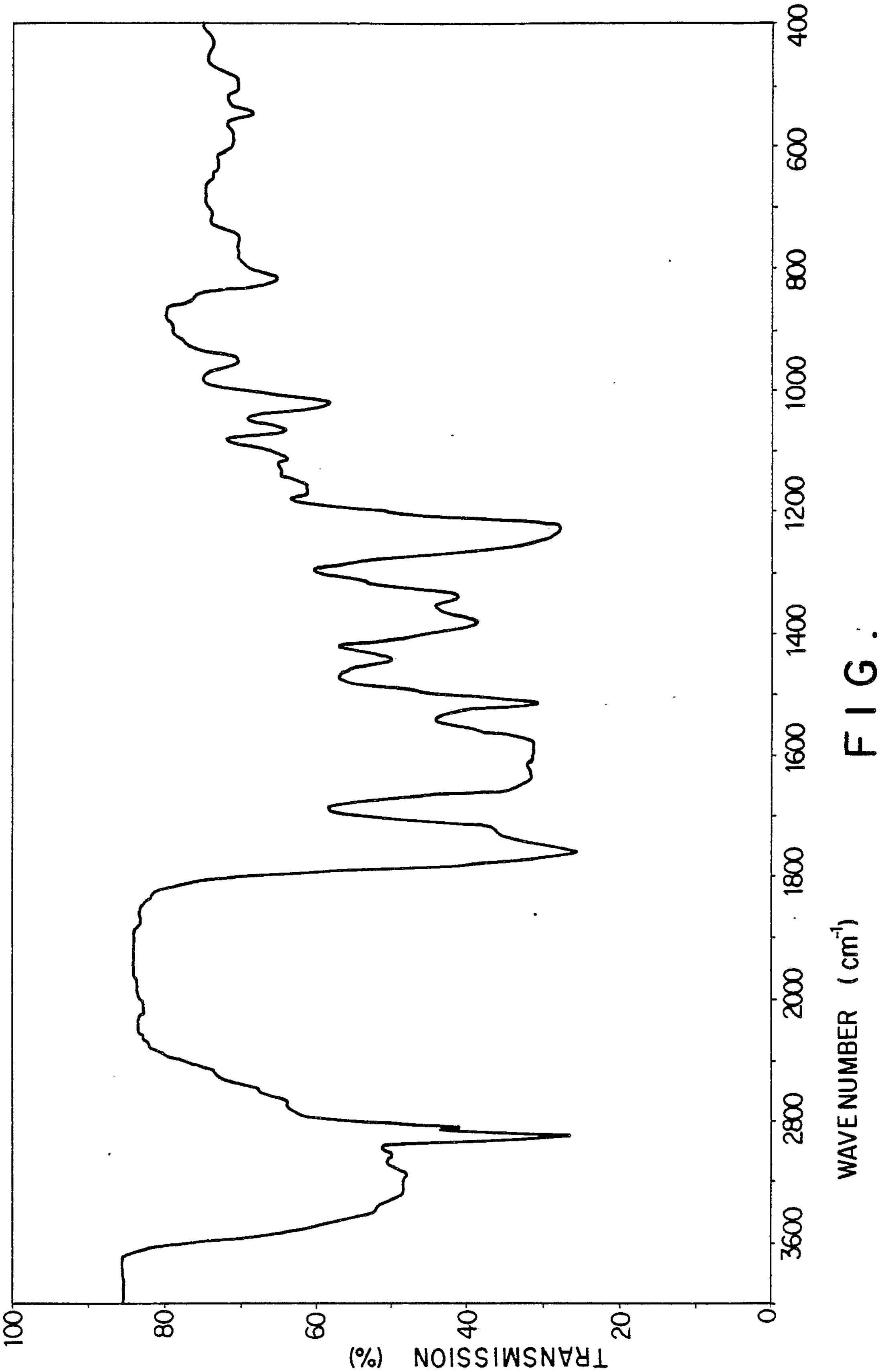
TRANSMISSION (%)
100
80
60
40
20
0
WAVE NUMBER (cm$^{-1}$)
3600
2800
2000
1800
1600
1400
1200
1000
800
600
400

FIG.